Europäisches Patentamt

European Patent Office

Office européen des brevets

(19)

(11) Veröffentlichungsnummer: **0 143 348**
**A1**

(12) **EUROPÄISCHE PATENTANMELDUNG**

(21) Anmeldenummer: **84113013.1**

(22) Anmeldetag: **29.10.84**

(51) Int. Cl.⁴: **A 61 F 5/04**

(30) Priorität: **31.10.83 DE 8331287 U**

(43) Veröffentlichungstag der Anmeldung: **05.06.85**
**Patentblatt 85/23**

(84) Benannte Vertragsstaaten: **AT BE CH DE FR GB IT LI NL**

(71) Anmelder: **Reinhold Hauber Strickwarenfabrik Gmbh & Co. KG, Sigmaringer Strasse 14, D-7440 Nürtingen (DE)**

(72) Erfinder: **Der Erfinder hat auf seine Nennung verzichtet**

(74) Vertreter: **Becker, Maria, Dipl.-Phys., Auf dem Haigst 29, D-7000 Stuttgart 70 (DE)**

(54) **Mittelhand-Daumenschiene.**

(57) Die Erfindung betrifft eine Mittelhand-Daumenschiene zur Ruhigstellung der Handwurzel zum Zweck einer therapeutischen Behandlung. Sie besteht aus einer Kappe (10), die in angelegtem Zustand die Handfläche und den Handrücken teilweise abdeckt, während sie einen rohrförmigen stirnseitig offenen Ansatz (12) zur Aufnahme des Daumens aufweist. Der am Handgelenk anliegende Teil (14a, 16a) ist in Form einer das Handgelenk umgreifenden elastischen Spange (14, 16) ausgebildet, die an ihren Enden mit einem Verschlußelement um das Handgelenk spannbar ist. Bei Verwendung von bei Wärmeeinwirkung plastifizierbarem Kunststoff läßt sich eine individuelle Anpassung der Bandage an Daumen und Hand erreichen.

Beschreibung:                    -2-                    0143348

Die Erfindung betrifft eine Mittelhand-Daumenschiene mit an dieser vorgesehenen Verschlusselementen zur Ruhigstellung der Handwurzel.

Der Erfindung liegt die Aufgabe zugrunde, ein therapeutisches Hilfsmittel zur Ruhigstellung der Handwurzel zu schaffen, wobei gleichzeitig Daumensattel- und Grundgelenk fixiert werden sollen. Trotz dieser Ruhigstellung soll aber die Greiffunktion des Daumens nicht beeinträchtigt werden.

Diese Aufgabe wird erfindungsgemäss durch eine Mittelhand-Daumenschiene gelöst, die gekennzeichnet ist durch eine im angelegten Zustand den Daumenballen und den Daumen mindestens bis zu dessen obersten Glied umfassende Kappe, mit einem den Daumen bis zu dem unteren Daumengelenk aufnehmenden rohrförmigen Ansatz, bei der der rohrförmige Ansatz und der entlang der äusseren Daumenfläche in den Handgelenkteil verlaufende Längsmittelbereich formsteif ausgebildet sind und bei der der am Handgelenk anliegende Teil das Handgelenk in Form einer elastischen Spange, die an ihren Enden mit einem Verschlusselement spannbar ist, umschliesst.

Der den Daumen aufnehmende rohrförmige Ansatz gewährleistet einen einwandfreien Halt der Kappe an der Hand, wobei zugleich das Daumensattel- und Grundgelenk ruhiggestellt sind, während durch die das Handgelenk umschliessende elastische Spange eine individuelle Anpassung an die Hand möglich ist.

Die Kappe bildet eine Bandage zur Teilimmobilisation der radialen Handwurzel, des Daumensattel- und Grundgelenkes, bei Sattelgelenk-Arthrose, bei Arthrose der radialen Mittelhand und des MTP 1, ulnarer Seitenbandläsion MTP 1 und Naviculare-Pseudarthrose.

Sofern die Kappe aus einem unter Wärmeeinwirkung plastifizierbaren Kunststoff, insbesondere einem unter dem Markennamen "Plexidur" oder "Hostalen" bekannten Kunststoff hergestellt wird, bietet sich die Möglichkeit, diese individuell gemäss der Anatomie der Hand des Patienten anmodellieren zu können, indem der Kunststoff erwärmt und entsprechend der Handform plastifiziert.wird.

Die Verschlusselemente dienen dazu, die Kappe fest an das Handgelenk anzulegen. Vorteilhaft bilden diese hierbei einen an sich bekannten Klettverschluss, der ein stufenloses Anpassen an den Handumfang gestattet.

Bei einer bevorzugten Ausführungsform bildet hierbei ein Verschlusselement einen an der Kappe, insbesondere an deren den Rücken des Handgelenkes abdeckenden Teil befestigtes, mit Haftelementen ausgestattetes Band, dem am anderen, das Handgelenk abdeckenden Kappenteil entsprechende Gegenhaftelemente zugeordnet sind.

In der Zeichnung ist ein Ausführungsbeispiel der Erfindung dargestellt. Es zeigen:

Fig. 1     eine schaubildliche Darstellung der Mittelhand-Daumenschiene, deren Verschlussband
           gelöst ist,

Fig. 2     eine schaubildliche Darstellung der Mittelhand-Daumenschiene in an einer Hand angelegtem Zustand sowie in einer Ansicht, wie
           sie sich am Handrücken bietet,

Fig. 3    eine schaubildliche Darstellung der an
          eine Hand angelegten Mittelhand-Daumenschiene in einer Ansicht, wie sie sich bei
          Betrachtung der Handinnenseite ergibt.

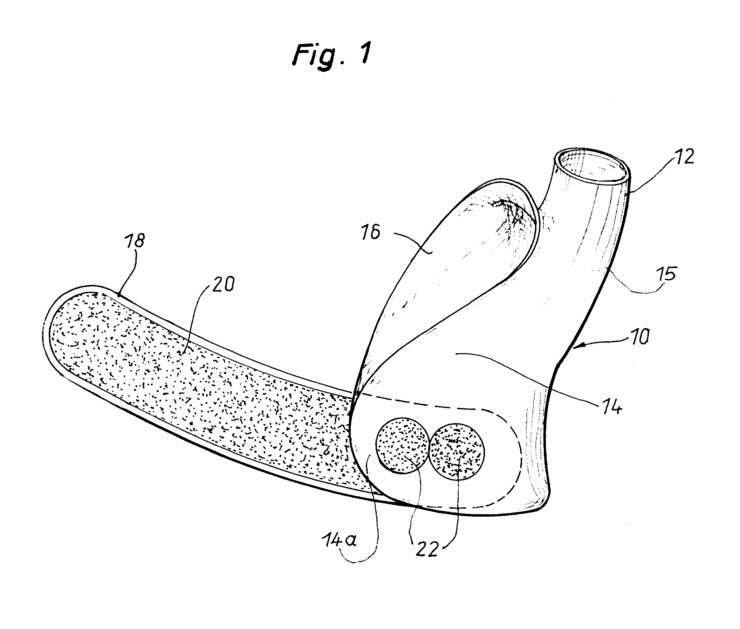

Die in Fig. 1 gezeigte Mittelhand-Daumenschiene
weist eine als Ganzes mit 10 bezeichnete Kappe auf,
die einen dünnwandigen Formkörper aus unter Wärmeeinwirkung plastifizierbarem, mechanisch besonders
festen Kunststoff bildet. Es kann sich hierbei um
Methacrylharz handeln.

An ihrem einen, gemäss Fig. 1 in Richtung der
Finger verlaufenden oberen Ende läuft die Kappe in
einen rohrförmigen Ansatz 12 aus, welcher zumindest
das untere Glied des Daumens der Hand aufnimmt, an
welche die Mittelhand-Daumenschiene angelegt werden
soll. Dieser rohrförmige Ansatz kann durch Kürzen
an die gewünschte Länge angepasst werden.

Das gezeigte Ausführungsbeispiel ist zum Anlegen an
der linken Hand ausgebildet. Sie ist hierzu im Abstand vom oberen Stirnende des rohrförmigen Ansatzes 12 in Art einer daumenseitig die betreffende
Hand umschliessenden Klammer gestaltet, deren beide
Klammerschenkel mit 14 und 16 bezeichnet sind.

Diese Klammerschenkel weisen in Richtung des Handgelenkes eine solche Verbreiterung 14a, 16a auf,
dass sie im angelegten Zustand der Kappe das Handgelenk in Art einer elastischen Spange zu dessen
Ruhigstellung umgreifen.

Der Längsmittelbereich 15 der Kappe 10, der in den rohrförmigen Ansatz 12 übergeht, ist formsteif und in Längsrichtung unelastisch ausgebildet, wodurch eine absolute Ruhigstellung der Daumenpartie bis zur Handwurzel erreicht wird. In Querrichtung dagegen ist die Kappe 10 elastisch und dadurch an die Hand- und Daumenfläche anschmiegbar. Sie fixiert somit die Daumenpartie ohne in die Haut des Trägers zu drücken.

An dem seine maximale Umfangsweite aufweisenden Bereich 16a des Klammerschenkels 16 ist ein Verschlussband 18 befestigt, das an seiner Innenseite Klettelemente 20 aufweist. Diesem sind am entsprechenden Endbereich des Klammerschenkels 14 an dessen Aussenseite Gegenklettelemente 22 zugeordnet, wodurch sich das Verschlussband zur optimalen Umschliessung des Handgelenkes am Klammerschenkel 14 festlegen lässt.

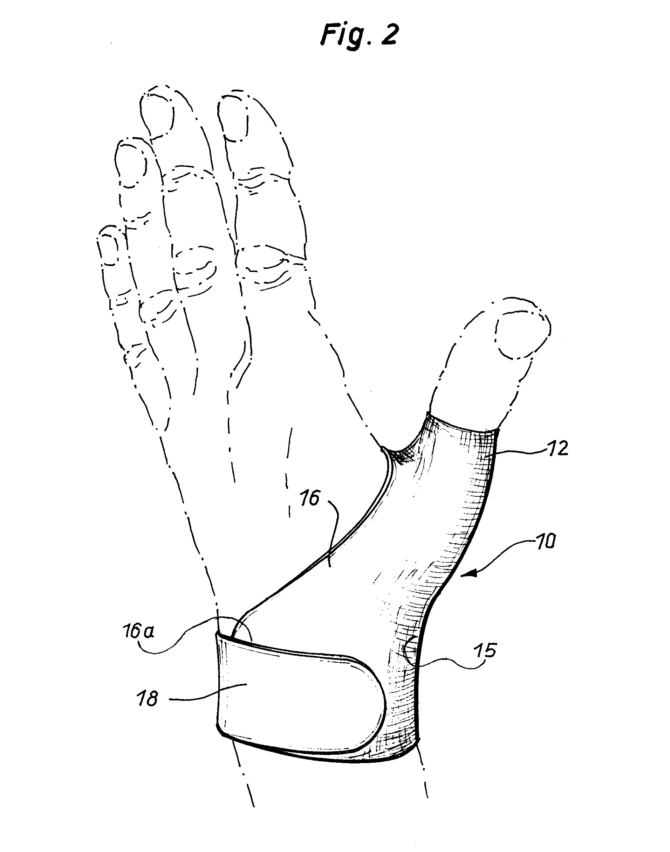

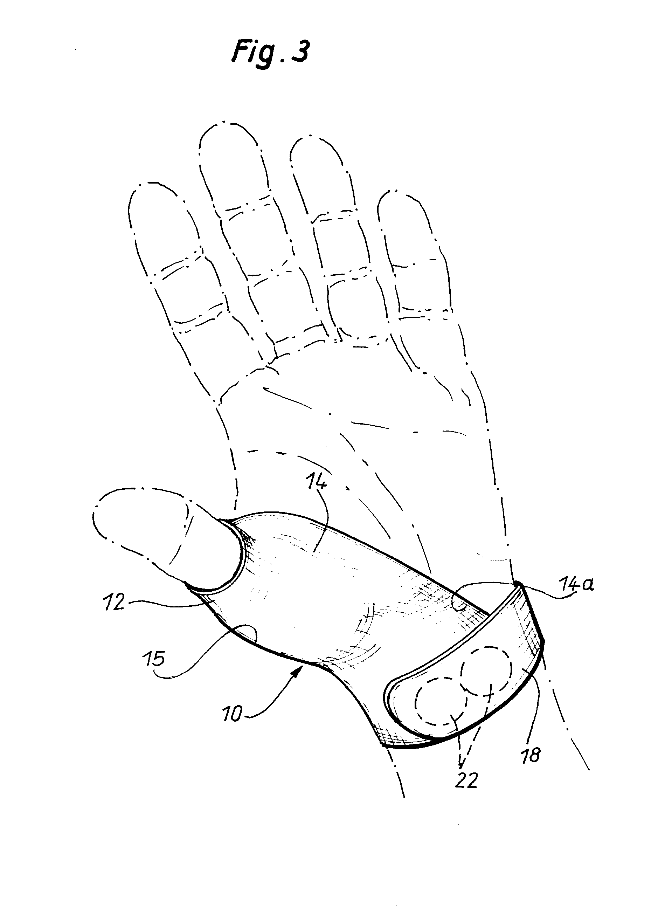

Wie aus den Fig. 2 und 3 ersichtlich ist, wird durch den rohrförmigen Ansatz 12 die Kappe der Mittelhand-Daumenschiene in Richtung Handgelenk an der Hand festgelegt, so dass ein einwandfreier Sitz derselben bei gleichzeitiger fester Handbandagierung gewährleistet ist.

Die Länge des Rohransatzes 12 kann auch derart gewählt sein, dass auch das obere Daumenglied fixiert wird, je nach gewünschter therapeutischer Wirkung.

Die Verwendung des einerseits biegesteifen, andererseits plastisch verformbaren Kunststoffes als Daumenbandage hat den Vorteil, dass das Material wasserfest und abwaschbar ist. Eine Abfütterung an der Innenseite ist leicht möglich.

Die Erfindung ermöglicht es, Bandagen für verschiedene Handgrössen zu schaffen, sofern beispielsweise drei oder vier unterschiedlich grosse Kappen bereitgehalten werden.

Patentansprüche

1. Mittelhand-Daumenschiene mit an dieser vorgesehenen Verschlusselementen zur Ruhigstellung der Handwurzel, gekennzeichnet durch eine in angelegtem Zustand den Daumenballen und den Daumen mindestens bis zu dessen obersten Glied umfassende Kappe (10) mit einem den Daumen bis zu dem unteren Daumengelenk aufnehmenden rohrförmigen Ansatz (12), bei der der rohrförmige Ansatz (12) und der entlang der äusseren Daumenfläche in den Handgelenkteil verlaufende Längsmittelbereich (15) formsteif ausgebildet sind und bei der der am Handgelenk anliegende Teil das Handgelenk in Form einer elastischen Spange (14a, 16a), die an ihren Enden mit einem Verschlusselement (18, 20, 22) spannbar ist, umschliesst.

2. Mittelhand-Daumenschiene nach Anspruch 1, dadurch gekennzeichnet, dass die Kappe (10) aus einem unter Wärmeeinwirkung plastifizierbaren Kunststoff, insbesondere "Hostalen" (eingetragenes Warenzeichen), besteht.

3. Mittelhand-Daumenschiene nach Anspruch 1 oder 2, dadurch gekennzeichnet, dass die Kappe (10) in Längsrichtung der Hand, d.h. in Richtung des rohrförmigen Ansatzes (12), unelastisch, jedoch in Querrichtung elastisch nachgiebig ausgebildet ist.

4. Mittelhand-Daumenschiene nach Anspruch 1 oder 2, dadurch gekennzeichnet, dass die Verschlusselemente (18, 20, 22) einen Klettverschluss bilden.

0143348

5. Mittelhand-Daumenschiene nach Anspruch 4, dadurch gekennzeichnet, dass ein Verschlusselement ein an der Kappe (10), insbesondere an deren den Rücken des Handgelenkes abdeckenden Teil, befestigtes, mit Haftelementen (18) ausgestattetes Verschlussband (20) ist, dem am anderen, das Handgelenk abdeckenden Kappenteil (14) entsprechende Gegenhaftelemente (22) zugeordnet sind.

## Fig. 1

0143348

# Fig. 2

0143348

## Fig.3

Europäisches
Patentamt

**EUROPÄISCHER RECHERCHENBERICHT**

0143348
Nummer der Anmeldung

EP 84 11 3013

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CH-A- 319 772 (ESTA) <br><br> * Seite 2, Zeilen 16-39, 65-81 * | 1,2 | A 61 F 5/04 |
| Y | | 4,5 | |
| | --- | | |
| X | US-A-2 523 606 (M.K. YOUNG) <br><br> * Spalte 2, Zeilen 3-29; Figuren 1-3 * | 1,2 | |
| | --- | | |
| X | US-A-1 471 948 (J.C. COX et al.) <br><br> * Seite 1, Zeilen 59-92; Figuren 1-4 * | 1,3 | |
| A | | 5 | **RECHERCHIERTE SACHGEBIETE (Int. Cl.4)** |
| | --- | | A 61 F <br> A 63 B <br> A 41 D |
| X,P | US-A-4 438 532 (A.F. CAMPANELLA) <br><br> * Insgesamt * | 1,2, 4 | |
| | --- | | |
| Y | DE-A-3 006 362 (E.W. PRINZ) <br><br> * Seite 7, Zeile 16 - Seite 9, Zeile 21; Figur * | 4,5 | |
| A | | 1,2 | |
| | --- | | |
| | -/- | | |

Der vorliegende Recherchenbericht wurde für alle Patentansprüche erstellt.

| Recherchenort | Abschlußdatum der Recherche | Prüfer |
|---|---|---|
| DEN HAAG | 06-02-1985 | WOLF C.H.S. |

EPA Form 1503 03 82

Europäisches
Patentamt

**EUROPÄISCHER RECHERCHENBERICHT**

0143348
Nummer der Anmeldung

EP 84 11 3013
Seite 2.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 005 615 (J.G. RHEE)<br><br>* Zusammenfassung; Figuren 9-11, 40,41 *<br><br>--- | 1-5 | |
| A | US-A-4 366 812 (R. NUZZO)<br><br>* Zusammenfassung; Figuren 2,7 *<br><br>--- | 1,3-5 | |
| A | DE-C- 587 234 (H. RUMMEL)<br><br>* Insgesamt *<br><br>--- | 3 | |
| A | US-A-3 050 053 (A.C. PECKHAM)<br><br>* Spalte 4, Zeile 43 - Spalte 5, Zeile 22; Figuren 5,6,9 *<br><br>--- | 1-3 | RECHERCHIERTE SACHGEBIETE (Int. Cl.4) |
| A | DE-C- 811 256 (A. FERSTL)<br><br>* Anspruch 1; Figuren *<br><br>--- | 3 | |
| A | US-A-3 327 703 (P.B. GAMM)<br><br>* Spalte 3, Zeilen 3-37; Spalte 5, Zeilen 4-57 *<br><br>--- | 3 | |
|  | -/- | | |

Der vorliegende Recherchenbericht wurde für alle Patentansprüche erstellt.

| Recherchenort | Abschlußdatum der Recherche | Prüfer |
|---|---|---|
| DEN HAAG | 06-02-1985 | WOLF C.H.S. |

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-4 161 175 (M. BENTELE)<br><br>* Zusammenfassung *<br><br>--- | 3 | |
| A | FR-A-2 181 157 (R. LEE)<br><br>* Insgesamt *<br><br>--- | 4,5 | |
| A | US-A-2 740 121 (R.K.F. SEIDEL)<br><br>* Insgesamt *<br><br>----- | 3 | |

RECHERCHIERTE
SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| DEN HAAG | 06-02-1985 | WOLF C.H.S. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82